# EUROPÄISCHE PATENTSCHRIFT

(11) **EP 2 809 760 B1**
(45) Veröffentlichungstag und Bekanntmachung des Hinweises auf die Patenterteilung: **20.03.2019**
(21) Anmeldenummer: 12801736.5
(22) Anmeldetag: 17.12.2012
(51) Int. Cl.: C11D 3/00, C11D 3/30, C11D 7/32, A61L 9/01, A61L 9/14, A01N 33/08

(54) **VERFAHREN ZUR VERMINDERUNG VON SCHLECHTGERÜCHEN**
PROCESS FOR REDUCING MALODOURS
PROCÉDÉ D'ATTÉNUATION DE MAUVAISES ODEURS

(30) Priorität: 01.02.2012 DE 102012201429
(43) Veröffentlichungstag der Anmeldung: 10.12.2014
(73) Patentinhaber: Henkel AG & Co. KGaA, 40589 Düsseldorf (DE)
(72) Erfinder: HUCHEL, Ursula, 50733 Köln (DE); WEYHE, Marc, 47802 Krefeld (DE); BARON, Lukas, 45307 Essen (DE); RITTLER, Frank, 40547 Düsseldorf (DE); BAUER, Andreas, 41564 Kaarst (DE)
(74) Vertreter: Viering, Jentschura & Partner mbB Patent- und Rechtsanwälte
(86) Internationale Anmeldenummer: PCT/EP2012/075719
(87) Internationale Veröffentlichungsnummer: WO 2013/113444

(56) Entgegenhaltungen:
- EP-A1- 1 787 689
- WO-A1-2010/142479
- US-A1- 2006 251 597
- DATABASE WPI Week 200707 Thomson Scientific, London, GB; AN 2007-062905 XP002693901, & JP 2006 320711 A (KAO CORP) 30. November 2006 (2006-11-30)
- DATABASE WPI Week 200841 Thomson Scientific, London, GB; AN 2008-G43077 XP002693902, & JP 2007 291540 A (KAO CORP) 8. November 2007 (2007-11-08)

## Beschreibung

Die vorliegende Erfindung betrifft ein Verfahren zum Abbau von Fehlgerüchen, vorzugsweise mit Blick auf die Behandlung harter und/oder weicher Oberflächen, insbesondere betrifft es den Abbau von Fehlgerüchen im Rahmen eines Textilbehandlungsverfahrens und ebenso den Abbau von Fehlgerüchen in der Raumluft.

Ein wichtiges Verbraucherbedürfnis, welches z.B. auch bei der Anwendung von Wasch-, Reinigungs- oder Pflegemitteln eine Rolle spielt, besteht in der Beseitigung oder zumindest in der Verminderung von Schlechtgerüchen (d.h. Fehlgerüchen) oder unerwünschten Gerüchen. Fehlgerüche gehen von bestimmten geruchsaktiven Verbindungen aus, welche auch als Stinkstoffe bezeichnet werden. Stinkstoffe sind übel riechende Verbindungen mit sogenannten kakosmophoren Gruppen, z.B. Schwefel-Derivate. Die Anwesenheit solcher Fehlgerüche führt in der Regel zu einer Beeinträchtigung des menschlichen Wohlbefindens, weshalb der Verbraucher die Auslöschung dieser Gerüche anstrebt. Allerdings werden die Fehlgerüche oftmals nicht ausgelöscht, sondern lediglich überdeckt. Dazu werden gewöhnlich Produkte eingesetzt, welche flüchtige, meist angenehm riechende Stoffe enthalten und welche bereits in kleinen Mengen üble Gerüche überdecken können.

Im Stand der Technik wird in der internationalen Patentanmeldung WO 2010/142479A1 der Einsatz bestimmter 2-Amino-1,3-propandiole und/oder substituierter 2-Amino-1,3-propandiole beschrieben, um Fehlgerüche, hervorgerufen durch die Anwesenheit von Aldehyden und/oder Ketonen, abzubauen. Die Verminderung von Fehlgerüchen, die auf Säuren, Thiolen, Sulfiden, Aminen, Aromaten, Alkohole und/oder Heteroaromaten zurückzuführen sind, wird dort nicht behandelt.

In den Patentveröffentlichungen EP 1 787 689, JP 2006320711 und JP 2007291540, wird der Einsatz bestimmter 2-Amino-1,3-propandiole und/oder substituierter 2-Amino-1,3-propandiole in Deodorants zur Bekämpfung von Schweißgerüchen beschrieben.

Aufgabe der vorliegenden Erfindung war es, dem Verbraucher eine Möglichkeit zu geben, eine Verminderung von Fehlgerüchen, die auf Säuren, Thiole, Sulfide, Amine, Aromaten, Alkohole und/oder Heteroaromaten zurückzuführen sind, herbeizuführen.

Die zuvor formulierte Aufgabe wird durch den Gegenstand der Erfindung gelöst, nämlich ein Verfahren zum Abbau von Fehlgerüchen, hervorgerufen durch die Anwesenheit von Säuren, Thiolen, Sulfiden, Aminen, Aromaten, Alkoholen und/oder Heteroaromaten, wobei 2-Amino-1,3-propandiol und/oder substituiertes 2-Amino-1,3-propandiol gemäß der Formel (I) 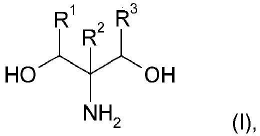 wobei in dieser Formel die Reste R¹, R² sowie R³, jeweils unabhängig voneinander, für Wasserstoff oder Kohlenwasserstoffreste stehen, welche acyclisch oder cyclisch, substituiert oder unsubstituiert, verzweigt oder unverzweigt sowie gesättigt oder ungesättigt sein können, und 0,1 bis 3 Gew.-% Riechstoffe, umfassend Riechstoff-Aldehyde und/oder Riechstoff-Ketone, und 5 bis 40 Gew.-% Tenside umfassend Anion- und/oder Niotensid im Rahmen eines Wasch- oder Reinigungsprozesses zugegeben werden, Gew.-% bezogen auf das gesamte Mittel. Die Kohlenwasserstoffreste im Sinne der Erfindung können grundsätzlich Heteroatome, wie z.B. Stickstoff-, Sauerstoff- oder Schwefelatome umfassen. Es kann sich bei den Kohlenwasserstoffresten im Sinne der Erfindung grundsätzlich auch um aromatische Kohlenwasserstoffreste handeln.

R¹ und R³ stehen insbesondere, jeweils unabhängig voneinander, für C₁₋₆-Alkylreste, vorzugsweise C₁₋₃-Alkylreste, oder für Wasserstoff. Besonders bevorzugte Reste R² sind neben Wasserstoff insbesondere Methyl-, Ethyl-, und Hydroxymethylreste. In einer bevorzugten Ausführungsform bedeuten R¹, R² und R³ jeweils Wasserstoff.

Bevorzugt im Sinne der Erfindung einsetzbar sind 2-Amino-2-(hydroxymethyl)propan-1,3-diol, 2-Amino-1,3- propandiol (Serinol), 2-Amino-2-methylpropan-1,3-diol (Methylserinol) sowie deren Mischungen.

Das 2-Amino-1,3-propandiol und/oder substituiertes 2-Amino-1,3-propandiol wird in einen Behälter mit Wasser oder einer Waschlauge gegeben, es kann direkt auf z.B. ein Waschgut bestehend aus vielen verschmutzten Hemden oder ähnlichem, gegeben werden.

Es konnte überraschend gefunden werden, dass das erfindungsgemäße Verfahren eine deutliche Verminderung bis hin zur Auslöschung von Schlecht- bzw. Fehlgerüchen bzw. unerwünschten Gerüchen, die auf die Anwesenheit von Säuren, Thiolen, Sulfiden, Aminen, Aromaten, Alkoholen und/oder Heteroaromaten zurückzuführen sind, ermöglicht.

Übelriechende Säuren, Thiole, Sulfide, Amine, Aromaten, Alkohole sowie Heteroaromaten sind dem Fachmann wohlbekannt, es handelt sich z.B. um Buttersäure, 1-Butanthiol, Dimethylsulfid, 3-Methyl-1H-indol, Tetrahydrothiophen usw.

Das erfindungsgemäße Verfahren ist insbesondere zur Minimierung oder Auslöschung von Fehlgerüchen mit Blick auf übelriechende Textilien, die z.B. nach sportlicher Aktivität resultieren erfolgreich einsetzbar. Gemäß der Erfindung wird das 2-Amino-1,3-propandiol und/oder substituierte 2-Amino-1,3-propandiol gemäß Formel (I) zusammen mit Riechstoffen eingesetzt, umfassend Riechstoff-Aldehyd und/oder Riechstoff-Keton. Riechstoff-Aldehyde sind diejenigen Riechstoffe, welche chemisch ein Aldehyd sind und welche vorteilhafterweise beim Menschen ein insbesondere angenehmes Geruchsempfinden auslösen. Riechstoff-Ketone sind diejenigen Riechstoffe, welche chemisch ein Keton sind und welche vorteilhafterweise beim Menschen ein insbesondere angenehmes Geruchsempfinden auslösen. Besonders geeignete Riechstoff-Aldehyde sowie Riechstoff-Ketone werden weiter unten exemplarisch aufgeführt.

Der Gehalt an Riechstoffen in dem erfindungsgemäß einsetzbaren Mittel beträgt 0,1 bis 3 Gew.-%, Gew.-% bezogen auf das gesamte Mittel. Der kombinierte Einsatz von 2-Amino-1,3-propandiol und/oder substituiertem 2-Amino-1,3-propandiol mit Riechstoffen, umfassend Riechstoff-Aldedyde und/oder Riechstoff-Ketone, Kombination mit Tensiden, ermöglicht eine ganz besonders gute, erfindungsgemäß angestrebte Verminderung von Fehlgerüchen.

Der Riechstoff-Aldehyd ist gemäß einer bevorzugten Ausführungsform der Erfindung ausgewählt aus Adoxal (2,6,10-Trimethyl-9-undecenal), Anisaldehyd (4-Methoxybenzaldehyd), Cymal (3-(4-Isopropyl-phenyl)-2-methylpropanal), Ethylvanillin, Florhydral (3-(3-isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Di-methyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)-propanal, 2-Methyl-4-(2,6,6-timethyl-2(1)-cyclo-hexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)-oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-car-boxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pen-tenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarbox-aldehyd, 7-Hy-droxy-3,7-dimethyl-octanal, trans-4-Decenal, 2,6-Nonadienal, para-Tolyl-acetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacet-aldehyd, 5,9-Di-methyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolace-taldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

Das Riechstoff-Keton ist gemäß einer bevorzugten Ausführungsform der Erfindung ausgewählt aus Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-lonon, beta-Ionon, gamma-Methyl-lonon, Fleuramon (2-heptylcyclopen-tanon), Dihydrojasmon, cis-Jasmon, iso-E-Super (1-(1,2,3,4,5,6,7,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldecahydro-2-naphton, Dimethyloctenon, Frescomenthe (2-butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-benzodioxol-5-yl) butan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amyl-cyclohexanon), 4-tert-butyl cyclohexanon, Delphon (2-pentyl-cyclopentanon), Muscon (CAS 541-91-3), Neobutenon (1-(5,5-dimethyl-1-cyclohexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on sowie Tetrameran (6,10-dimethylundecen-2-on).

Grundsätzlich gilt, dass weitere optionale Riechstoffe, die in dem erfindungsgemäßen Verfahren zusätzlich eingesetzt werden können, keinen besonderen Beschränkungen unterworfen sind, sofern sie dazu beitragen, eine ansprechende Duftnote zu erzeugen. So können einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclo-hexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 bis 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Es können auch natürliche Riechstoffgemische eingesetzt werden, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Weitere herkömmliche Riechstoffe, die im Rahmen der vorliegenden Erfindung zusätzlich eingesetzt werden können, sind beispielsweise die ätherischen Öle wie Angelikawurzelöl, Anisöl, Arnikablütenöl, Basilikumöl, Bayöl, Champacablütenöl, Edeltannenöl, Edeltannen-zapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxy-acetophenon, Methyl-n-amylketon, Methylanthranilsäuremethylester, p-Methylaceto-phenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylalde-hyd, Nonylalkohol, n-Octylaldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtaldehyd, Zimtalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Gemäß der Erfindung erfolgt die Zugabe des 2-Amino-1,3-propandiols und/oder des substituierten 2-Amino-1,3-propandiol gemäß Formel (I) im Rahmen eines Waschprozesses. Dabei wird das 2-Amino-1,3-propandiol und/oder substituierte 2-Amino-1,3-propandiol insbesondere als Bestandteil eines, riechstoffhaltigen, Waschmittel eingesetzt. Hierbei erfolgt eine besonders gute Verminderung des Schlechtgeruches.

Kein Gegenstand der Erfindung ist ein Reinigungs- oder Pflegeverfahren unter Einsatz eines Reinigungsmittels, eines Air-Care-Produktes oder eines Raumluftverbesserers, umfassend 2-Amino-1,3-propandiol und/oder substituiertes 2-Amino-1,3-propandiol gemäß der Formel (I) 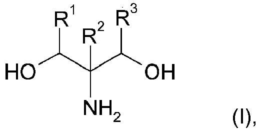 wobei in dieser Formel die Reste R¹, R² sowie R³, jeweils unabhängig voneinander, für Wasserstoff oder Kohlenwasserstoffreste, welche acyclisch oder cyclisch, substituiert oder unsubstituiert, verzweigt oder unverzweigt sowie gesättigt oder ungesättigt sein können, stehen, vorzugsweise in Mengen von 0,001 bis 5 Gew.-% und 0,1 bis 3 Gew.-% Riechstoffe, umfassend Riechstoff-Aldehyde und/oder Riechstoff-Ketone, Gew.-% bezogen auf das gesamte Mittel, zum Abbau von Fehlgerüchen, hervorgerufen durch die Anwesenheit von Säuren, Thiolen, Sulfiden, Aminen, Aromaten, Alkoholen und/oder Heteroaromaten.

Die genannten Kohlenwasserstoffreste können grundsätzlich Heteroatome, wie z.B. Stickstoff-, Sauerstoff- oder Schwefelatome umfassen. Es kann sich bei den genannten Kohlenwasserstoffresten grundsätzlich auch um aromatische Kohlenwasserstoffreste handeln. R¹ und R³ stehen in Formel (I) insbesondere, jeweils unabhängig voneinander, für C₁₋₆-Alkylreste, vorzugsweise C₁₋₃-Alkylreste, oder für Wasserstoff. Besonders bevorzugte Reste R² sind neben Wasserstoff insbesondere Methyl-, Ethyl-, und Hydroxymethylreste. In einer bevorzugten Ausführungsform bedeuten R¹, R² und R³ jeweils Wasserstoff.

Bevorzugt einsetzbar sind 2-Amino-2-(hydroxymethyl)propan-1,3-diol, 2-Amino-1,3- propandiol (Serinol), 2-Amino-2-methylpropan-1,3-diol (Methylserinol) sowie deren Mischungen.

Es konnte überraschend gefunden werden, dass ein solches Wasch-, Reinigungs- oder Pflegeverfahren unter Einsatz eines entsprechenden Wasch- oder Reinigungsmittels, eines Air-Care-Produktes oder eines Raumluftverbesserers eine deutliche Verminderung von Schlechtgerüchen bzw. unerwünschten Gerüchen, die auf die Anwesenheit von Säuren, Thiolen, Sulfiden, Aminen, Aromaten, Alkoholen und/oder Heteroaromaten zurückzuführen sind, ermöglicht. Außerdem konnte insgesamt ein verbessertes Dufterlebnis erreicht werden, insbesondere im Zusammenhang mit der Textilbehandlung.

Weiterhin ist bevorzugt, dass das eingesetzte Waschmittel vor der Anwendung in fester Form vorliegt, vorzugsweise in Pulverform.

Geeignete Waschmittel in fester Form, wie vorzugsweise in Pulverform oder auch in Granulatform oder in Gestalt von Pressformkörpern, z.B. Tabletten, sind insbesondere für die Anwendung in wässrigen Systemen vorgesehen, z.B. für den Einsatz in einer Waschlauge.

Allerdings kann das eingesetzte Mittel vor der Anwendung auch in flüssiger Form vorliegen, vorzugsweise in Gelform. Dies entspricht ebenfalls einer bevorzugten Ausführungsform der Erfindung. Flüssige Mittel können auch direkt angewendet werden, z.B. durch Auftragen auf ein übelriechendes Objekt, ggf. können sie vorher verdünnt werden. Ebenso ist die Anwendung in wässrigen Systemen möglich.

Wenn das eingesetzte Waschmittel insbesondere zumindest 10 Gew.-% Tensid enthält, umfassend Aniontensid und/oder Niotensid, so liegt eine weitere bevorzugte Ausführungsform der Erfindung vor. Eine sinnvolle Obergrenze für Tensid kann z.B. 30 Gew.-% oder 20 Gew.-% liegen, Gew.-% jeweils bezogen auf das gesamte Mittel. So kann eine besonders gute, erfindungsgemäß angestrebte Verminderung von Fehlgerüchen bzw. unerwünschten Gerüchen bewirkt werden.

Handelt es sich bei dem zuvor beschriebenen Verfahren um ein Textilreinigungs- oder - konditionierverfahren, bei welchem das zu reinigende Textil einer Textilwäsche unterworfen wird, vorzugsweise in einer automatischen Waschmaschine, insbesondere bei Temperaturen im Bereich von 15 bis 60°C, noch vorteilhafter 15 bis 40°C, so liegt wiederum eine bevorzugte Ausführungsform der Erfindung vor.

Ein weiterer Gegenstand der Erfindung ist die Verwendung von Waschmitteln enthaltend Amino-1,3-propandiol und/oder substituiertem 2-Amino-1,3-propandiol gemäß der Formel (I) und 0,1 bis 3 Gew.-% Riechstoffen, umfassend Riechstoff-Aldehyde und/oder Riechstoff-Ketone, und 5-40 Gew.% Tenside umfassend Anion- und/oder Niotensid zum Abbau von Fehlgerüchen, hervorgerufen durch die Anwesenheit von Säuren, Thiolen, Sulfiden, Aminen, Aromaten, Alkoholen und/oder Heteroaromaten.

Nach einer weiteren bevorzugten Ausführungsform weist das erfindungsgemäß einsetzbare Mittel, wenigstens eine, vorzugsweise mehrere, aktive Komponenten, insbesondere wasch- und/oder reinigungsaktive Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silikonöle, Soil release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das erfindungsgemäß einsetzbare Mittel in Gew.-%, wenn nicht anders angegeben, auf das Gesamtgewicht des erfindungsgemäß einsetzbaren Mittels.

Die Mengen der einzelnen Inhaltsstoffe in den erfindungsgemäß einsetzbaren Mitteln orientieren sich jeweils am Einsatzzweck der betreffenden Mittel und der Fachmann ist mit den Größenordnungen der einzusetzenden Mengen der Inhaltsstoffe vertraut oder kann diese der zugehörigen Fachliteratur entnehmen. Je nach Einsatzzweck der erfindungsgemäßen Mittel wird man z.B. den Tensidgehalt höher oder niedriger wählen. Üblicherweise kann z.B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäß einsetzbaren Mittel enthalten anionische Tenside, nichtionische Tenside und deren Gemische, aber zusätzlich auch kationische Tenside

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer MethylGruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind z.B. Benzalkoniumchlorid (N Alkyl-N,N dimethyl-benzylammoniumchlorid), Benzalkon B (m,p-Dichlorbenzyl-dimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈- bis C₂₂-Alkylresten, insbesondere C₁₂- bis C₁₄-Alkyl-benzyl-dimethylammoniumchlorid.

Unter Esterquats sollen hier vorzugsweise Verbindungen der allgemeinen Formel IV, 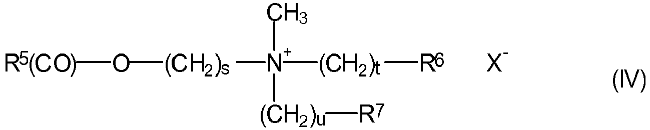 verstanden werden, in der R⁵ für einen Alkyl- oder Alkenylrest mit 12 bis 22 Kohlenstoffatomen und 0, 1, 2 oder 3 Doppelbindungen, R⁶ und R⁷ unabhängig voneinander für H, OH oder O(CO)R⁵, s, t und u jeweils unabhängig voneinander für den Wert 1, 2 oder 3 und X- für ein Anion, insbesondere Halogenid, Methosulfat, Methophosphat oder Phosphat sowie Mischungen aus diesen, steht. Bevorzugt sind Verbindungen, die für R⁶ die Gruppe O(CO)R⁵ und für R⁵ einen Alkylrest mit 16 bis 18 Kohlenstoffatomen enthalten. Besonders bevorzugt sind Verbindungen, bei denen R⁷ zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Gruppen aufweisen, sind die Acylgruppen bevorzugt, deren korrespondierende Fettsäuren eine Jodzahl zwischen 5 und 80, vorzugsweise zwischen 10 und 60 und insbesondere zwischen 15 und 45 aufweisen und/oder die ein cis/trans-Isomerenverhältnis (in Mol-%) von größer als 30 : 70, vorzugsweise größer als 50 : 50 und insbesondere größer als 70 : 30 haben. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart® bekannten Produkte der Firma Cognis Deutschland GmbH beziehungsweise die unter der Bezeichnung Rewoquat® bekannten Produkte des Herstellers Goldschmidt-Witco.

Tenside sind gewünschtenfalls in den erfindungsgemäß einsetzbaren Mittel in Mengenanteilen von vorzugsweise 5 bis 50 Gew.-%, insbesondere von 8 bis 30 Gew.-%, enthalten. Insbesondere in Wäschenachbehandlungsmitteln werden vorzugsweise bis zu 30 Gew.-%, insbesondere 5 bis 15 Gew.-% Tenside, unter diesen bevorzugt wenigstens anteilsweise Kationtenside, eingesetzt.

Ein erfindungsgemäß einsetzbares Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättigten Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃- bis C₈-Carbonsäure und vorzugsweise von einer C₃- bis C₄-Monocarbonsäure, insbesondere von (Meth)-acrylsäure ab. Das zweite saure Monomer beziehungsweise dessen Salz kann ein Derivat einer C₄- bis C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, z.B. von C₁- bis C₄-Carbonsäuren, mit Vinylalkohol darstellen. Bevorzugte Polymere enthalten dabei 60 bis 95 Gew.-%, insbesondere 70 bis 90 Gew.-% (Meth)acrylsäure bzw. (Meth)acrylat, besonders bevorzugt Acrylsäure bzw. Acrylat, und Maleinsäure bzw. Maleinat sowie 5 bis 40 Gew.-%, vorzugsweise 10 bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁- bis C₄-Alkylrest, oder einem aromatischen Rest, der sich vorzugsweise von Benzol oder Benzol-Derivaten ableitet, substituiert ist. Bevorzugte Terpolymere enthalten dabei 40 bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 bis 30 Gew.-%, vorzugsweise 15 bis 25 Gew.-% Methallylsulfonsäure bzw. Methallylsulfonat und als drittes Monomer 15 bis 40 Gew.-%, vorzugsweise 20 bis 40 Gew.-% eines Kohlenhydrats. Dieses Kohlenhydrat kann dabei z.B. ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative Molekülmasse zwischen 1 000 und 200 000, vorzugsweise zwischen 200 und 50 000 und insbesondere zwischen 3 000 und 10 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 bis 8 Gew.-% enthalten sein. Mengen nahe der genannten Obergrenze werden vorzugsweise in pastenförmigen oder flüssigen, insbesondere wasserhaltigen, erfindungsgemäß einsetzbaren Mitteln eingesetzt. Erfindungsgemäß einsetzbare Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäß einsetzbaren Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisitikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäß einsetzbaren Mitteln eingesetzt werden. In einer weiteren bevorzugten Ausführungsform erfindungsgemäß einsetzbarer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäß einsetzbarer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in den erfindungsgemäß einsetzbaren Mitteln vorzugsweise in Mengen bis zu 60 Gew.-%, insbesondere von 5 bis 40 Gew.-%, enthalten. Erfindungsgemäß einsetzbare Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wässriger Lösungen, die 3 bis 10 Gew.-% Wasserstoffperoxid enthalten, eingesetzt. Falls ein erfindungsgemäß einsetzbares Mittel Bleichmittel, wie vorzugsweise Persauerstoffverbindungen, enthält, sind diese in Mengen von vorzugsweise bis zu 50 Gew.-%, insbesondere von 5 bis 30 Gew.-%, vorhanden. Der Zusatz geringer Mengen bekannter Bleichmittelstabilisatoren wie z.B. von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, z.B. N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 bis 10 Gew.-%, insbesondere 2 bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere Mangan-, Eisen-, Cobalt-, Ruthenium- oder Molybdän-Salenkomplexe und deren N-Analogverbindungen, Mangan-, Eisen-, Cobalt-, Ruthenium- oder Molybdän-Carbonylkomplexe, Mangan-, Eisen-, Cobalt-, Ruthenium-, Molybdän-, Titan-, Vanadium- und Kupfer-Komplexe mit stickstoffhaltigen Tripod-Liganden, Cobalt-, Eisen-, Kupfer- und Ruthenium-Amminkomplexe. Kombinationen aus Bleichaktivatoren und Übergangsmetall-Bleichkatalysatoren können ebenfalls eingesetzt werden. Bleichverstärkende Übergangsmetallkomplexe, insbesondere mit den Zentralatomen Mn, Fe, Co, Cu, Mo, V, Ti und/oder Ru, können in üblichen Mengen, vorzugsweise in einer Menge bis zu 1 Gew.-%, insbesondere von 0,0025 bis 0,25 Gew.-% und besonders bevorzugt von 0,01 bis 0,1 Gew.-%, jeweils bezogen auf gesamtes Mittel, eingesetzt werden.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind gewünschtenfalls in den erfindungsgemäß einsetzbaren Mitteln in Mengen vorzugsweise nicht über 5 Gew.-%, insbesondere von 0,2 bis 2 Gew.-%, enthalten.

Die Mittel können als optische Aufheller z.B. Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören z.B. Organopolysiloxane und deren Gemische mit mikrofeiner, gegebenenfalls silanierter Kieselsäure sowie Paraffinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffinhaltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäß einsetzbaren Mittel, das diese öl- und fettlösende Komponente enthält, gewaschen wurde. Zu den bevorzugten öl- und fettlösenden Komponenten zählen z.B. nicht-ionische Celluloseether wie Methylcellulose und Methylhydroxypropylcellulose mit einem Anteil an MethoxyGruppen von 15 bis 30 Gew.-% und an Hydroxypropoxy-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 bis 2 Gew.-%, insbesondere 0,1 bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone mit Molgewichten von 15 000 bis 50 000 wie auch Polyvinylpyrrolidone mit Molgewichten über 1 000 000, insbesondere von 1 500 000 bis 4 000 000, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist zum Einsatz in erfindungsgemäß einsetzbaren Mitteln vorzugsweise eine durchschnittliche Molmasse im Bereich von 10 000 bis 60 000, insbesondere im Bereich von 25 000 bis 50 000 auf. Unter den Copolymeren sind solche aus Vinylpyrrolidon und Vinylimidazol im Molverhältnis 5:1 bis 1:1 mit einer durchschnittlichen Molmasse im Bereich von 5 000 bis 50 000, insbesondere 10 000 bis 20 000 bevorzugt.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, z.B. Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, z.B. in Mengen von 0,1 bis 5 Gew.-%, bezogen auf die Mittel, eingesetzt.

Zu den in den erfindungsgemäß einsetzbaren Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäß einsetzbaren Mitteln vorzugsweise in Mengen von nicht über 30 Gew.-%, insbesondere von 6 bis 20 Gew.-%, vorhanden.

Zur Einstellung eines gewünschten, sich durch die Mischung der übrigen Komponenten nicht von selbst ergebenden pH-Werts können die erfindungsgemäß einsetzbaren Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäß einsetzbaren Mitteln vorzugsweise nicht über 20 Gew.-%, insbesondere von 1,2 bis 17 Gew.-%, enthalten.

Die Herstellung fester erfindungsgemäß einsetzbaren Mittel bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäß einsetzbarer Mittel mit erhöhtem Schüttgewicht, insbesondere im Bereich von 650 bis 950 g/l, ist ein einen Extrusionsschritt aufweisendes Verfahren bevorzugt. Die Herstellung flüssiger erfindungsgemäß einsetzbarer Mittel bereitet ebenfalls keine Schwierigkeiten und kann ebenfalls in bekannter Weise erfolgen.

Gemäß einer bevorzugten Ausführungsform kann die erfindungsgemäße Lehre dazu eingesetzt werden, den Parfümanteil in Waschmitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können. In diesem Zusammenhang sind vor allem z.B. Handwaschmittel zu nennen.

Ein bevorzugtes erfindungsgemäß einsetzbares festes, insbesondere pulverförmiges Waschmittel kann neben den erfindungsgemäß einzusetzenden Bestandteilen (also 2-Amino-1,3-propandiol und/oder substituiertes 2-Amino-1,3-propandiol sowie Riechstoffen umfassend Riechstoff-Aldehyde und/oder Riechstoff-Ketone sowie Anion- und oder Niotenside) insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 30 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 15 Gew.-%
- Gerüststoffe, wie z.B. Zeolith, Polycarboxylat, Natriumcitrat, in Mengen von z.B. 0 bis 70 Gew.-%, vorteilhafterweise 5 bis 60 Gew.-%, vorzugsweise 10 bis 55 Gew.-%, insbesondere 15 bis 40 Gew.-%,
- Alkalien, wie z.B. Natriumcarbonat, in Mengen von z.B. 0 bis 35 Gew.-% vorteilhafterweise 1 bis 30 Gew.-%, vorzugsweise 2 bis 25 Gew.-%, insbesondere 5 bis 20 Gew.-%,
- Bleichmittel, wie z.B. Natriumperborat, Natriumpercarbonat, in Mengen von z.B. 0 bis 30 Gew.-% vorteilhafterweise 5 bis 25 Gew.-%, vorzugsweise 10 bis 20 Gew.-%,
- Korrosionsinhibitoren, z.B. Natriumsilicat, in Mengen von z.B. 0 bis 10 Gew.-%, vorteilhafterweise 1 bis 6 Gew.-%, vorzugsweise 2 bis 5 Gew.-%, insbesondere 3 bis 4 Gew.-%,
- Stabilisatoren, z.B. Phosphonate, vorteilhafterweise 0 bis1 Gew.-%,
- Schauminhibitor, z.B. Seife, Siliconöle, Paraffine vorteilhafterweise 0 bis 4 Gew.-%, vorzugsweise 0,1 bis 3 Gew.-%, insbesondere 0,2 bis 1 Gew.-%,
- Enzyme, z.B. Proteasen, Amylasen, Cellulasen, Lipasen, vorteilhafterweise 0 bis 2 Gew.-%, vorzugsweise 0,2 bis 1 Gew.-%, insbesondere 0,3 bis 0,8 Gew.-%,
- Vergrauungsinhibitor, z.B. Carboxymethylcellulose, vorteilhafterweise 0 bis 1 Gew.-%,
- Verfärbungsinhibitor, z.B. Polyvinylpyrrolidon-Derivate, vorzugsweise 0 bis 2 Gew.-%,
- Stellmittel, z.B. Natriumsulfat, vorteilhafterweise 0 bis 20 Gew.-%,
- Optische Aufheller, z.B. Stilben-Derivat, Biphenyl-Derivat, vorteilhafterweise 0 bis 0,4 Gew.-%, insbesondere 0,1 bis 0,3 Gew.-%,
- weitere Duftstoffe,
- ggf. Wasser,
- ggf. Seife,
- ggf. Bleichaktivatoren,
- ggf. Cellulosederivate,
- ggf. Schmutzabweiser,
Gew.-% jeweils bezogen auf das gesamte Mittel.

In einer anderen bevorzugten Ausführungsform der Erfindung liegt das Waschmittel in flüssiger Form vor, vorzugsweise in Gelform. Bevorzugte flüssige Waschmittel haben Wassergehalte von z.B. 10 bis 95 Gew.-%, vorzugsweise 20 bis 80 Gew.-% und insbesondere 30 bis 70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäß einsetzbares flüssiges, insbesondere gelförmiges Waschmittel kann neben den erfindungsgemäß einzusetzenden Bestandteilen (also 2-Amino-1,3-propandiol und/oder substituiertes 2-Amino-1,3-propandiol sowie Riechstoffen umfassend Riechstoff-Aldehyde und/oder Riechstoff-Ketone sowie Anion- und oder Niotenside) insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 40 Gew.-%,
- Nichtionische Tenside, wie vorzugsweise Fettalkoholpolyglycolether, Alkylpolyglucosid, Fettsäureglucamid z.B. in Mengen von vorzugsweise 0,5 bis 25 Gew.-%,
- Gerüststoffe, wie z.B. Zeolith, Polycarboxylat, Natriumcitrat, vorteilhafterweise 0 bis 15 Gew.-%, vorzugsweise 0,01 bis 10 Gew.-%, insbesondere 0,1 bis 5 Gew.-%,
- Schauminhibitor, z.B. Seife, Siliconöle, Paraffine, in Mengen von z.B. 0 bis 10 Gew.-%, vorteilhafterweise 0,1 bis 4 Gew.-%, vorzugsweise 0,2 bis 2 Gew.-%, insbesondere 1 bis 3 Gew.-%,
- Enzyme, z.B. Proteasen, Amylasen, Cellulasen, Lipasen, in Mengen von z.B. 0 bis 3 Gew.-%, vorteilhafterweise 0,1 bis 2 Gew.-%, vorzugsweise 0,2 bis 1 Gew.-%, insbesondere 0,3 bis 0,8 Gew.-%,
- Optische Aufheller, z.B. Stilben-Derivat, Biphenyl-Derivat, in Mengen von z.B. 0 bis 1 Gew.-%, vorteilhafterweise 0,1 bis 0,3 Gew.-%, insbesondere 0,1 bis 0,4 Gew.-%,
- weitere Duftstoffe,
- ggf. Stabilisatoren,
- Wasser,
- ggf. Seife, in Mengen von z.B. 0 bis 25 Gew.-%, vorteilhafterweise 1 bis 20 Gew.-%, vorzugsweise 2 bis 15 Gew.-%, insbesondere 5 bis 10 Gew.-%,
- ggf. Lösungsmittel (vorzugsweise Alkohole), vorteilhafterweise 0 bis 25 Gew.-%, vorzugsweise 1 bis 20 Gew.-%, insbesondere 2 bis 15 Gew.-%, Gew.-% jeweils bezogen auf das gesamte Mittel.

Ein bevorzugter nicht erfindungsgemäß einsetzbarer flüssiger Weichspüler kann neben den erfindungsgemäß einzusetzenden Bestandteilen (also 2-Amino-1,3-propandiol und/oder substituiertes 2-Amino-1,3-propandiol sowie Riechstoffen umfassend Riechstoff-Aldehyde und/oder Riechstoff-Ketone) insbesondere noch Komponenten enthalten, die ausgewählt sind aus den folgenden:
- Kationische Tenside, wie insbesondere Esterquats, z.B. in Mengen von 5 bis 30 Gew.-%,
- Cotenside, wie z.B. Glycerolmonostearat, Stearinsäure, Fettalkohole, Fettalkoholethoxylate, z.B. in Mengen von 0 bis 5 Gew.-%, vorzugsweise 0,1 bis 4 Gew.-%,
- Emulgatoren, wie z.B. Fettaminethoxylate, z.B. in Mengen von 0 bis 4 Gew.-%, vorzugsweise 0,1 bis 3 Gew.-%,
- weitere Duftstoffe,
- Farbstoffe, vorzugsweise im ppm-Bereich,
- Stabilisatoren, vorzugsweise im ppm-Bereich,
- Lösemittel, wie insbesondere Wasser, in Mengen von vorzugsweise 60 bis 90 Gew.-%, Gew.-% jeweils bezogen auf das gesamte Mittel.

### Beispiel:

Ein Versuchsbehälter von 35 l Rauminhalt war mit einer 1 cm großen Öffnung zum Anschluss eines Schlauchs versehen, mit dem eine olfaktorische Kontrolle der darin befindlichen Atmosphäre möglich war. Über eine seitlich Klappe in Bodennähe konnte der Behälter rasch geöffnet und mit Untersuchungsmedien (siehe unten) bestückt werden.

Der Versuch fand bei Raumtemperatur unter Normaldruck statt. Es wurden zwei identisch aufgebaute Behälter eingesetzt. In die gereinigten und geruchsneutralen Behälter wurde jeweils eine Petri-Schale mit 5 ml einer 10%igen Fehlgeruchslösung A (in Polyethylenglykol 2000) platziert. Diese Fehlgeruchslösung A (Rezeptur siehe unten) dient zur Nachstellung eines Fehlgeruches, welcher für Toilettenräume üblich ist. Nach einer Stunde wurde die Petri Schale entfernt. Nach 30 min ergab die olfaktorische Kontrolle der beiden Behälter jeweils einen intensiven üblen Toiletten-Fehlgeruch. Mittels einer Sprühflasche wurde eine Prüflösung (siehe unten) fein verteilt in einen der Versuchsbehälter eingebracht. Dabei wurde jeweils ein Sprühstoß mit einem Volumen von 0,1 ml erzeugt. Als Lösungsmittel wurde Wasser verwendet. Die Lösungen hatten einen 10 %igen Aktivstoffgehalt. Unmittelbar nach dem Sprühstoß sowie in den in der Tabelle aufgeführten Zeitabständen wurde eine Beurteilung der Geruchsintensität in der Kammer durchgeführt. Die Skala der Geruchsintensität reicht von stark (Wert 10) bis nicht wahrnehmbar (Wert 0). Die Beurteilung wurde von geruchlich geschulten Menschen vorgenommen.

Ergebnisse:

| | Intensität nach 0 h | Intensität nach 1 h | Intensität nach 2 h | Intensität nach 4 h | Intensität nach 8 h |
|---|---|---|---|---|---|
| Kammer mit Serinollösung beaufschlagt | 10 | 3 | 1 | 1 | 0 |
| Kammer ohne Beaufschlagung mit einer Serinollösung | 10 | 6 | 6 | 5 | 4 |
| Kammer mit Methylserinol-lösung beaufschlagt | 10 | 4 | 2 | 1 | 0 |

Die Zugabe von Serinol bzw. Methylserinol in das System ermöglichte also einen Abbau des Fehlgeruches.

Rezeptur der eingesetzten Fehlgeruchslösung A (Toilettengeruch)

| | |
|---|---|
| 3-Methylbutansäure | 20 Gew.-% |
| n-Butansäure | 20 Gew.-% |
| n-Hexansäure | 20 Gew.-% |
| 2,3-Benzopyrrol | 20 Gew.-% |
| 4-Methyl-2,3-Benzopyrrol | 10 Gew.-% |
| Ammoniaklösung (25%ig in Wasser) | 10 Gew.-% |

## Patentansprüche

1. Verfahren zum Abbau von Fehlgerüchen, hervorgerufen durch die Anwesenheit von Säuren, Thiolen, Sulfiden, Aminen, Aromaten, Alkoholen und/oder Heteroaromaten, **dadurch gekennzeichnet, dass** ein Waschmittel enthaltend 2-Amino-1,3-propandiol und/oder substituiertes 2-Amino-1,3-propandiol gemäß der Formel (I) 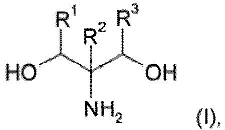 wobei in dieser Formel die Reste R¹, R² sowie R³, jeweils unabhängig voneinander, für Wasserstoff oder Kohlenwasserstoffreste stehen, welche acyclisch oder cyclisch, substituiert oder unsubstituiert, verzweigt oder unverzweigt sowie gesättigt oder ungesättigt sein können, 0,1 bis 3 Gew.-% Riechstoffe, umfassend Riechstoff-Aldehyde und/oder Riechstoff-Ketone, und 5 bis 40 Gew.-% Tensid umfassend Anion- und/oder Niotensid, im Rahmen eines Waschprozesses zugegeben wird, wobei die Gew.-% bezogen auf das gesamte Mittel sind.

2. Verfahren gemäß Anspruch 1, **dadurch gekennzeichnet, dass** das 2-Amino-1,3-propandiol und/oder das substituierte 2-Amino-1,3-propandiol gemäß Formel (I) zusammen mit Riechstoffen eingesetzt wird, insbesondere umfassend
(a) Riechstoff-Aldehyd ausgewählt aus Adoxal (2,6,10-Trimethyl-9-undecenal), Anisaldehyd (4-Methoxybenzaldehyd), Cymal (3-(4-lsopropylphenyl)-2-methylpropanal), Ethylvanillin, Florhydral (3-(3-isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)-oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Di-methyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal,
und/oder
(b) Riechstoff-Keton ausgewählt aus Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-lonon, beta-lonon, gamma-Methyl-lonon, Fleuramon (2-heptylcyclopentanon), Dihydrojasmon, cis-Jasmon, iso-E-Super (1-(1,2,3,4,5,6,7,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3-methyl-5-propyl-2-cyclohexenon), 6-Isopropyldecahydro-2-naphton, Dimethyloctenon, Frescomenthe (2-butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-benzodioxol-5-yl) butan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amyl-cyclohexanon), 4-tert-butyl cyclohexanon, Delphon (2-pentyl-cyclopentanon), Muscon (CAS 541-91-3), Neobutenon (1-(5,5-dimethyl-1-cyclohexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on sowie Tetrameran (6,10-dimethylundecen-2-on).

3. Waschverfahren unter Einsatz eines Waschmittels, umfassend 2-Amino-1,3-propandiol und/oder substituiertes 2-Amino-1,3-propandiol gemäß der Formel (I) 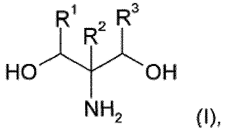 wobei in dieser Formel die Reste R¹, R² sowie R³, jeweils unabhängig voneinander, für Wasserstoff oder Kohlenwasserstoffreste, welche acyclisch oder cyclisch, substituiert oder unsubstituiert, verzweigt oder unverzweigt sowie gesättigt oder ungesättigt sein können, stehen, in Mengen von 0,1 bis 3 Gew.-% Riechstoffe, umfassend Riechstoff-Aldehyde und/oder Riechstoff-Ketone, und 5 bis 40 Gew.-% Tensid umfassend Anion- und/oder Niotensid, wobei die Gew.-% bezogen auf das gesamte Mittel sind, zum Abbau von Fehlgerüchen, hervorgerufen durch die Anwesenheit von Säuren, Thiolen, Sulfiden, Aminen, Aromaten, Alkoholen und/oder Heteroaromaten.

4. Verfahren nach Anspruch 3, **dadurch gekennzeichnet, dass** das eingesetzte Waschmittel vor der Anwendung in fester Form vorliegt, vorzugsweise in Pulverform.

5. Verfahren nach Anspruch 3, **dadurch gekennzeichnet, dass** das eingesetzte Waschmittel vor der Anwendung in flüssiger Form vorliegt, vorzugsweise in Gelform.

6. Verfahren nach einem der Ansprüche 4 oder 5, **dadurch gekennzeichnet, dass** das eingesetzte Waschmittel zumindest 10 Gew.-% Aniontensid und/oder Niotensid enthält.

7. Verfahren nach einem der Ansprüche 4 bis 6, **dadurch gekennzeichnet, dass** es sich um ein Textilreinigungs- oder -konditionierverfahren handelt, bei welchem das zu reinigende Textil einer Textilwäsche unterworfen wird, vorzugsweise in einer automatischen Waschmaschine, insbesondere bei Temperaturen im Bereich von 15 bis 60 °C, vorteilhafter 15 bis 40 °C.

8. Verwendung von Waschmitteln enthaltend Amino-1,3-propandiol und/oder substituiertem 2-Amino-1,3-propandiol gemäß der Formel (I), 0,1 bis 3 Gew.-% Riechstoffen, umfassend Riechstoff-Aldehyde und/oder Riechstoff-Ketone und 5 bis 40 Gew.-% Tensid umfassend Anion- und/oder Niotensid zum Abbau von Fehlgerüchen, hervorgerufen durch die Anwesenheit von Säuren, Thiolen, Sulfiden, Aminen, Aromaten, Alkoholen und/oder Heteroaromaten.

## Claims

1. A method for reducing malodors caused by the presence of acids, thiols, sulfides, amines, aromatics, alcohols and/or heteroaromatics, **characterized in that** a washing agent containing 2-amino-1,3-propanediol and/or substituted 2-amino-1,3-propanediol according to formula (I) 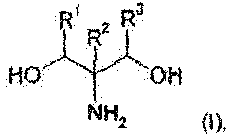 wherein, in this formula, the groups R¹, R² and R³, each independently of one another, represent hydrogen or hydrocarbon groups, which can be acyclic or cyclic, substituted or unsubstituted, branched or unbranched and saturated or unsaturated, from 0.1 to 3 wt.% of odorants comprising odorant aldehydes and/or odorant ketones, and from 5 to 40 wt.% of surfactant comprising anionic and/or non-ionic surfactant, is added during a washing process, the wt.% being based on the total agent.

2. The method according to claim 1, **characterized in that** the 2-amino-1,3-propanediol and/or the substituted 2-amino-1,3-propanediol according to formula (I) is used together with odorants, in particular comprising
(a) odorant aldehyde selected from adoxal (2,6,10-trimethyl-9-undecenal), anisaldehyde (4-methoxybenzaldehyde), cymene (3-(4-isopropylphenyl)-2-methylpropanal), ethylvanillin, Florhydral (3-(3-isopropylphenyl)-butanal), helional (3-(3,4-methylenedioxyphenyl)-2-methylpropanal), heliotropin, hydroxycitronellal, lauraldehyde, Lyral (3- and 4-(4-hydroxy-4-methylpentyl)-3-cyclohexene-1-carboxaldehyde), methylnonylacetaldehyde, lilial (3-(4-tert-butylphenyl)-2-methylpropanal), phenylacetaldehyde, undecylenic aldehyde, vanillin, 2,6,10-trimethyl-9-undecenal, 3-dodecene-1-al, alpha-n-amylcinnamaldehyde, melonal (2,6-dimethyl-5-heptenal), 2,4-dimethyl-3-cyclohexene-1-carboxaldehyde (Triplal), 4-methoxybenzaldehyde, benzaldehyde, 3-(4-tert-butylphenyl)-propanal, 2-methyl-3-(para-methoxyphenyl)propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decylidene-8)-butanal, octahydro-4,7-methano-1H-indenecarboxaldehyde, 3-ethoxy-4-hydroxybenzaldehyde, para-ethyl-alpha, alpha-dimethyl hydrocinnamaldehyde, alpha-methyl-3,4-(methylenedioxy)-hydrocinnamaldehyde, 3,4-methylenedioxybenzaldehyde, alpha-n-hexylcinnamaldehyde, m-cymene-7-carboxaldehyde, alpha-methylphenylacetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexene-3-carboxaldehyde, 4-(4-hydroxy-4-methylpentyl)-3-cyclohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanoindane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyloctanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindane-1-carboxaldehyde, 2-methyloctanal, alpha-methyl-4-(1-methylethyl)benzolacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]-hept-5-ene-2-carbaldehyde, 9-decenal, 3-methyl-5-phenyl-1-pentanal, methylnonyl acetaldehyde, hexanal and trans-2-hexenal, and/or
(b) odorant ketone selected from methyl-beta-naphthylketone, musk indanone (1,2,3,5,6,7-hexahydro-1,1,2,3,3-pentamethyl-4H-inden-4-one), tonalide (6-acetyl-1,1,2,4,4,7-hexamethyltetralin), alpha-damascones, beta-damascones, delta-damascones, iso-damascones, damascenones, methyl dihydrojasmonate, menthone, carvone, camphor, Koavone (3,4,5,6,6-pentamethylhept-3-en-2-one), fenchone, alpha-ionone, beta-ionone, gamma-methyl ionone, fleuramone (2-heptylcyclopentanone), dihydrojasmone, cis-jasmone, Iso E Super (1-(1,2,3,4,5,6,7,8-octahydro-2,3,8,8-tetramethyl-2-naphthalenyl)-ethan-1-one (and isomers)), methyl cedrenyl ketone, acetophenone, methylacetophenone, para-methoxyacetophenone, methyl-beta-naphtylketone, benzylacetone, benzophenone, para-hydroxyphenyl butanone, celery ketone (3-methyl-5-propyl-2-cyclohexenone), 6-isopropyldecahydro-2-naphtone, dimethyloctenone, Frescomenthe (2-butan-2-yl-cyclohexan-1-one), 4-(1-ethoxyvinyl)-3,3,5,5-tetramethylcyclohexanone, methylheptenone, 2-(2-(4-methyl-3-cyclohexen-1-yl)propyl)-cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)-indanone, 4-damascol, Dulcinyl (4-(1,3-benzodioxol-5-yl) butan-2-one), hexalone (1-(2,6,6-trimethyl-2-cyclohexene-1-yl)-1,6-heptadien-3-one), isocyclemone E (2-acetonaphthone-1,2,3,4,5,6,7,8-octahydro-2,3,8,8-tetramethyl), methylnonylketone, methylcyclocitrone, methyl lavender ketone, orivone (4-tert-amyl-cyclohexanone), 4-tert-butylcyclohexanone, Delphone (2-pentylcyclopentanone), muscone (CAS 541-91-3), Neobutenone (1-(5,5-dimethyl-1-cyclohexenyl)pent-4-en-1-one), plicatone (CAS 41724-19-0), Veloutone (2,2,5-trimethyl-5-pentylcyclopentan-1-one), 2,4,4,7-tetramethyl-oct-6-en-3-one, and tetramerane (6,10-dimethylundecen-2-one).

3. A washing method using a washing agent, comprising 2-amino-1,3-propanediol and/or substituted 2-amino-1,3-propanediol according to formula (I) 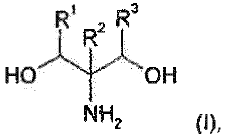 wherein, in this formula, the groups R¹, R² and R³, each independently of one another, represent hydrogen or hydrocarbon groups, which can be acyclic or cyclic, substituted or unsubstituted, branched or unbranched and saturated or unsaturated, in amounts of from 0.1 to 3 wt.% of odorants comprising odorant aldehydes and/or odorant ketones, and from 5 to 40 wt.% of surfactant comprising anionic and/or non-ionic surfactant, the wt.% being based on the total agent, in order to reduce malodors caused by the presence of acids, thiols, sulfides, amines, aromatics, alcohols and/or heteroaromatics.

4. The method according to claim 3, **characterized in that** the washing agent used is present in solid form, preferably in powder form, before application.

5. The method according to claim 3, **characterized in that** the washing agent used is present in liquid form, preferably in gel form, before application.

6. The method according to any one of claims 4 or 5, **characterized in that** the washing agent used contains at least 10 wt.% of anionic surfactant and/or non-ionic surfactant.

7. The method according to any one of claims 4 to 6, **characterized in that** it is a textile cleaning or conditioning method in which the textile to be washed is subjected to a textiles wash, preferably in an automatic washing machine, in particular at temperatures in the range of from 15 to 60 °C, preferably from 15 to 40 °C.

8. The use of washing agents containing amino-1,3-propanediol and/or substituted 2-amino-1,3-propanediol according to formula (I), from 0.1 to 3 wt.% of odorants comprising odorant aldehydes and/or odorant ketones and from 5 to 40 wt.% of surfactant comprising anionic and/or non-ionic surfactant in order to reduce malodors caused by the presence of acids, thiols, sulfides, amines, aromatics, alcohols and/or heteroaromatics.

## Revendications

1. Procédé pour la réduction des mauvaises odeurs provoquées par la présence d'acides, de thiols, de sulfures, d'amines, de composés aromatiques, d'alcools et/ou de composés hétéroaromatiques, **caractérisé en ce qu'**un détergent contenant du 2-amino-1,3-propanediol et/ou du 2 amino-1,3-propanediol substitué selon la formule (I) 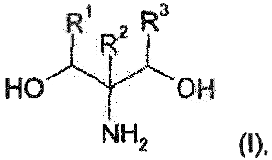 les radicaux R¹, R² et R³ représentant, dans cette formule, chacun indépendamment l'un de l'autre, l'hydrogène ou des radicaux hydrocarbonés pouvant être acycliques ou cycliques, substitués ou non substitués, ramifiés ou non ramifiés, ainsi que saturés ou insaturés, de 0,1 à 3 % en poids de parfums, comprenant des aldéhydes parfumés et/ou des cétones parfumées, et de 5 à 40 % en poids de tensioactif comprenant un tensioactif anionique et/ou non ionique,
est ajouté dans le cadre d'un processus de lavage, le pourcentage en poids se rapportant au poids total de l'agent.

2. Procédé selon la revendication 1, **caractérisé en ce que** le 2-amino-1,3-propanediol et/ou le 2-amino-1,3-propanediol substitué selon la formule (I) est utilisé avec des parfums, comprenant notamment
(a) un aldéhyde parfumé choisi parmi l'adoxal (2,6,10-triméthyl-9-undécénal), l'anisaldéhyde (4-méthoxybenzaldéhyde), le cymal (3-(4-isopropylphényl)-2-méthylpropanal), l'éthylvanilline, le florhydral (3-(3-isopropylphényl)butanal), l'hélional (3-(3,4-méthylènedioxyphényl)-2-méthylpropanal), l'héliotropine, l'hydroxycitronellal, le lauraldéhyde, le lyral (3- et 4-(4-hydroxy-4-méthylpentyl)-3-cyclohexène-1-carboxaldéhyde), le méthylnonylacétaldéhyde, le lilial (3-(4-tert-butylphényl)-2-méthylpropanal), le phénylacétaldéhyde, l'undécylènealdéhyde, la vanilline, le 2,6,10-triméthyl-9-undécénal, le 3-dodécén-1-al, l'alpha-n-amylcinnamaldéhyde, le mélonal (2,6-diméthyl-5-hepténal), le 2,4-diméthyl-3-cyclohexène-1-carboxaldéhyde (triplai), le 4-méthoxybenzaldéhyde, le benzaldéhyde, le 3-(4-tert-butylphényl)-propanal, le 2-méthyl-3-(para-méthoxyphényl)propanal, le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)-oxy]acétaldéhyde, le 4-isopropylbenzylaldéhyde, le 1,2,3,4,5,6,7,8-octahydro-8,8-diméthyl-2-naphtalaldéhyde, le 2,4-diméthyl-3-cyclohexène-1-carboxaldéhyde, le 2-méthyl-3-(isopropylphényl)propanal, le 1-décanal, le 2,6-diméthyl-5-hepténal, le 4-(tricyclo[5.2.1,0(2,6)]-décylidène-8)-butanal, l'octahydro-4,7-méthane-1H-indécarboxaldéhyde, le 3-éthoxy-4-hydroxybenzaldéhyde, le para-éthyl-alpha, alpha-diméthylhydrocinnamaldéhyde, méthylènedioxybenzaldéhyde, l'alpha-n-hexylcinnamaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexènecarboxaldéhyde, le 1-dodécanal, le 2,4-diméthylcyclohexène-3-carboxaldéhyde, le 4-(4-hydroxy-4-méthylpentyl)-3-cyclohexène-1 -carboxaldéhyde, le 7-méthoxy-3,7-diméthyloctan-1-al, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5- ou 6-méthoxyhexahydro-4,7-méthanindane-1- ou -2-carboxaldéhyde, le 3,7-diméthyloctan-1-al, le 1-undécanal, le 10-undécén-1-al, le 4-hydroxy-3-méthoxybenzaldéhyde, le 1-méthyl-3-(4-méthylpentyl)-3-cyclohexènecarboxaldéhyde, le 7-hydroxy-3,7-diméthyl-octanal, le trans-4-décénal, le 2,6-nonadiénal, le para-tolylacétaldéhyde, le 4-méthylphénylacétaldéhyde, le 2-méthyl-4-(2,6,6-triméthyl-1-cyclohexén-1-yl)-2-buténal, l'ortho-méthoxycinnamaldéhyde, le 3,5,6-triméthyl-3-cyclohexènecarboxaldéhyde, le 3,7-diméthyl-2-méthylén-6-octénal, le phénoxyacétaldéhyde, le 5,9-diméthyl-4,8-décadiénal, le péonyaldéhyde (6,10-diméthyl-3-oxa-5,9-undécadién-1-al), l'hexahydro-4,7-méthanindane-1-carboxaldéhyde, le 2-méthyloctanal, l'alpha-méthyl-4-(1-méthyléthyl)benzèneacétaldéhyde, le 6,6-diméthyl-2-norpinène-2-propionaldéhyde, le para-méthylphénoxyacétaldéhyde, le 2-méthyl-3-phényl-2-propén-1-al, le 3,5,5-triméthylhexanal, l'hexahydro-8,8-diméthyl-2-naphtaldéhyde, le 3-propyl-bicyclo[2.2.1]-hept-5-ène-2-carbaldéhyde, le 9-décénal, le 3-méthyl-5-phényl-1-pentanal, le méthylnonylacétaldéhyde, l'hexanal ainsi que le trans-2-hexénal,
et/ou
(b) une cétone parfumée choisi parmi la méthyl-bêta-naphtylcétone, la muskindanone (1,2,3,5,6,7-hexahydro-1,1,2,3,3-pentaméthyl-4H-indén-4-one), la tonalide (6-acétyl-1,1,2,4,4,7-hexaméthyltétraline), l'alpha-damascone, la bêta-damascone, la delta-damascone, l'iso-damascone, la damascénone, le méthyldihydrojasmonate, la menthone, la carvone, le camphre, la koavone (3,4,5,6,6-pentaméthylhept-3-én-2-one), la fenchone, l'alpha-ionone, la bêta-ionone, la gamma-méthyl-ionone, le fleuramone (2-heptylcyclopentanone), la dihydrojasmone, la cis-jasmone, l'iso-E-Super (1-(1,2,3,4,5,6,7,8-octahydro-2,3,8,8-tétraméthyl-2-naphtalényl)-éthan-1-one (et ses isomères)), la méthylcédrénylcétone, l'acétophénone, la méthylacétophénone, la para-méthoxyacétophénone, la méthyl-bêta-naphtylcétone, la benzylacétone, la benzophénone, la para-hydroxyphénylbutanone, la céleri-cétone(3-méthyl-5-propyl-2-cyclohexénone), la 6-isopropyldécahydro-2-naphtone, la diméthylocténone, la Frescomenthe (2-butan-2-yl-cyclohexan-1-one), la 4-(1-éthoxyvinyl)-3,3,5,5-tétraméthylcyclohexanone, la méthylhepténone, la 2-(2-(4-méthyl-3-cyclohexén-1-yl)propyl)cyclopentanone, la 1-(p-menthén-6(2)yl)-1-propanone, la 4-(4-hydroxy-3-méthoxyphényl)-2-butanone, le 2-acétyl-3,3-diméthylnorbornane, la 6,7-dihydro-1,1,2,3,3-pentaméthyl-4(5H)-indanone, le 4-damascol, la dulcinyl(4-(1,3-benzodioxol-5-yl)butan-2-one), l'hexalone (1-(2,6,6-triméthyl-2-cyclohexén-1-yl)-1,6-heptadié-3-one), l'isocyclémone E (2-acétonaphtone-1,2,3,4,5,6,7,8-octahydro-2,3,8,8-tétraméthyle), la méthylnonylcétone, la méthylcyclocitrone, la méthyllavandecétone, l'orivone (4-tert-amyl-cyclohexanone), le 4-tert-butyl-cyclohexanone, la delphone (2-pentyl-cyclopentanone), la muscone (CAS 541-91-3), la néobuténone (1-(5,5-diméthyl-1-cyclohexényl)pent-4-én-1-one), la plicatone (CAS 41724-19-0), la véloutone (2,2,5-triméthyl-5-pentylcyclopentan-1-one), la 2,4,4,7-tétraméthyl-oct-6-én-3-one ainsi que le tétramérane (6,10-diméthylundécén-2-one).

3. Procédé de lavage utilisant un détergent comprenant du 2-amino-1,3-propanediol et/ou du 2-amino-1,3-propanediol substitué selon la formule (I) 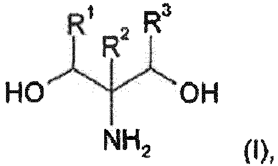 les radicaux R¹, R² et R³ représentant, dans cette formule, chacun indépendamment l'un de l'autre, l'hydrogène ou des radicaux hydrocarbonés pouvant être acycliques ou cycliques, substitués ou non substitués, ramifiés ou non ramifiés, et saturés ou insaturés, en des quantités de 0,1 à 3 % en poids de parfums, comprenant des aldéhydes parfumés et/ou des cétones parfumées, et de 5 à 40 % en poids de tensioactif comprenant un tensioactif anionique et/ou non ionique, les pourcentages en poids se rapportant à l'ensemble de l'agent, pour la réduction des mauvaises odeurs provoquées par la présence d'acides, de thiols, de sulfures, d'amines, de composés aromatiques, d'alcools et/ou de composés hétéroaromatiques.

4. Procédé selon la revendication 3, **caractérisé en ce que** le détergent utilisé avant l'utilisation est présent sous forme solide, de préférence sous forme de poudre.

5. Procédé selon la revendication 3, **caractérisé en ce que** le détergent utilisé est présent avant l'utilisation sous forme liquide, de préférence sous forme de gel.

6. Procédé selon l'une des revendications 4 ou 5, **caractérisé en ce que** le détergent utilisé contient au moins 10 % en poids de tensioactif anionique et/ou de tensioactif non ionique.

7. Procédé selon l'une des revendications 4 à 6, **caractérisé en ce qu'**il s'agit d'un procédé de nettoyage ou de conditionnement de textile, le textile à nettoyer étant soumis à un lavage de textile, de préférence dans une machine à laver automatique, en particulier à des températures comprises entre 15 et 60 °C, de manière davantage préférée entre 15 et 40 °C.

8. Utilisation de détergents contenant de l'amino-1,3-propanediol et/ou du 2-amino-1,3-propanediol substitué selon la formule (I), de 0,1 à 3 % en poids de parfums, comprenant des aldéhydes parfumés et/ou des cétones parfumées et de 5 à 40 % en poids de tensioactif comprenant un tensioactif anionique et/ou non ionique pour la réduction des mauvaises odeurs provoquées par la présence d'acides, de thiols, de sulfures, d'amines, de composés aromatiques, d'alcools et/ou de composés hétéroaromatiques.
